# NEUE EUROPÄISCHE PATENTSCHRIFT

(11) **EP 1 259 207 B2**
(45) Veröffentlichungstag und Bekanntmachung des Hinweises auf die Entscheidung über den Einspruch: **12.01.2011**
(45) Hinweis auf die Patenterteilung: 21.12.2005
(21) Anmeldenummer: 01925367.3
(22) Anmeldetag: 02.03.2001
(51) Int. Cl.: A61F 13/15, A61L 15/42, A61L 15/00

(54) **ABSORBIERENDER HYGIENEARTIKEL ZUM EINMALIGEN GEBRAUCH**
SINGLE-USE ABSORBENT SANITARY ARTICLE
ARTICLE D'HYGIENE ABSORBANT, A USAGE UNIQUE

(30) Priorität: 02.03.2000 DE 10010269; 02.03.2000 DE 10010268
(43) Veröffentlichungstag der Anmeldung: 27.11.2002
(73) Patentinhaber: Paul Hartmann Aktiengesellschaft, 89522 Heidenheim (DE)
(72) Erfinder: MALOWANIEC, Krzysztof, D., 89522 Heidenheim (DE); MANGOLD, Rainer, 89542 Herbrechtingen (DE); WURSTER, Thomas, 89522 Heidenheim (DE)
(74) Vertreter: Friz, Oliver
(86) Internationale Anmeldenummer: PCT/EP2001/002386
(87) Internationale Veröffentlichungsnummer: WO 2001/064154

(56) Entgegenhaltungen:
- EP-A- 0 418 493
- WO-A-98/56430
- WO-A1-95/10996
- WO-A1-99/47089
- WO-A2-98/56430
- US-A- 3 676 242
- US-A- 3 704 198
- US-A- 3 715 251
- US-A- 4 554 292
- US-A- 5 061 259
- US-A- 5 147 345
- US-A- 5 318 554
- US-A- 5 849 850
- US-A- 5 859 077
- US-A- 6 019 871

## Beschreibung

Die Erfindung betrifft einen absorbierenden Hygieneartikel zum einmaligen Gebrauch, insbesondere eine Windel, Damenbinde oder Inkontinenzvorlage, mit einem wenigstens zweischichtigen Saugkörper, der eine im Gebrauch des Artikels körperzugewandte Flüssigkeitsaufnahme-, -verteiler- und -zwischenspeicherschicht und eine auf deren körperabgewandter Seite vorgesehene Speicherschicht mit einem Anteil von wenigstens 50 Gew.-% an superabsorbierenden Polymermaterialien umfaßt.

Derartige Hygieneartikel sind in großer Anzahl bekannt, beispielsweise aus WO-A-97/34557. Des weiteren betrifft die Erfindung ein Verfahren zum Herstellen einer Flüssigkeitsaufnahme-, -verteiler- und -zwischenspeicherschicht im Zuge der Herstellung eines Hygieneartikels der genannten Art.

Bei gattungsgemäßen Hygieneartikeln dient der hohe Anteil an superabsorbierenden Polymermaterialien in der im Gebrauch körperabgewandt angeordneten Speicherschicht zur dauerhaften Speicherung der aufgenommenen Flüssigkeit, indem die superabsorbierenden Polymermaterialien wässrige Flüssigkeit binden und dabei in einen gelartigen Zustand überführt werden. Da die hierfür erforderliche Zeit gegenüber der Abgabezeit der Flüssigkeit während des Wasserlassens durch den Träger des Hygieneartikels verhältnismäßig lang ist, wird in bekannter Weise eine Flüssigkeitsaufnahme-, -verteiler- und -zwischenspeicherschicht vorgesehen, welche auf der körperzugewandten Seite der Speicherschicht angeordnet wird. Die Aufgabe dieser Flüssigkeitsaufnahme-, -verteiler- und -zwischenspeicherschicht ist es, für das schwallartige Auftreffen von Flüssigkeit eine hinreichend große Aufnahmerate bereitzustellen, um zu verhindern, daß Flüssigkeit in Quer- oder Längsrichtung über den Hygieneartikel rinnt und nach außerhalb des Hygieneartikels gelangt, was dessen Funktionsfähigkeit beeinträchtigen würde. Des weiteren soll die Flüssigkeitsaufnahme-, -verteiler- und -zwischenspeicherschicht die verhältnismäßig konzentriert auftreffende Flüssigkeit in andere Bereiche des Saugkörpers leiten, um die dort vorhandene Flüssigkeitsabsorptionskapazität ausnutzen zu können. Es soll also eine Flüssigkeitsverteilung innerhalb der körperzugewandten Verteilerschicht, aber auch eine kurzzeitige Zwischenspeicherung und anschließende Abgabe an die darunter angeordnete Speicherschicht erreicht werden.

Es wurden bereits Anstrengungen unternommen, für die Flüssigkeitsaufnahme-, -verteiler- und -zwischenspeicherschicht Materialien einzusetzen, die auch bei Einnässung, insbesondere bei wiederholter Einnässung, die vorstehend erwähnte Funktion der körperzugewandten Schicht zumindest weitestgehend unbeeinträchtigt von der Einnässung ausüben können. Es wurden vorzugsweise intravernetzte Zellulosefasern, die auch als "curled fiber" bezeichnet werden, für den Einsatz in der körperzugewandten Verteilerschicht eingesetzt. Eine aus intravernetzten Zellulosefasern bestehende oder solche Fasern zu einem beträchtlichen Anteil umfassende Verteilerschicht behält nämlich auch nach Einnässung ein verhältnismäßig großes Flüssigkeitsaufnahmevolumen und fällt im Gegensatz zu einer aus natürlichen nichtvernetzten Zellulosefasern gebildeten Schicht bei Einnässung nicht in sich zusammen ("wet collaps").

Hiervon ausgehend liegt der vorliegenden Erfindung die Aufgabe zugrunde, einen absorbierenden Hygieneartikel der gattungsgemäßen Art dahingehend zu verbessern, daß seine Flüssigkeitsaufnahme-, -verteiler- und -zwischenspeicherschicht auch ohne beträchtliche Anteile an intravernetzten Zellulosefasern eine wenigstens ebenso gute Flüssigkeitsaufnahme-, -verteiler- und Zwischenspeichercharakteristik aufweist wie die vorstehend erwähnten Hygieneartikel.

Diese Aufgabe wird durch einen gattungsgemäßen Hygieneartikel mit den Merkmalen des Anspruch 1 gelöst.

Es zeigte sich, daß auftreffende wässrige Flüssigkeit sehr rasch in die durch Extrusion unter Expansion des Treibmittels gebildete offenporige geschäumte Struktur eindringen und dort zwischengespeichert werden kann. Die extrudierte Schicht stellt ein entsprechend dem Schäumungsgrad, der größer als 50 % und in besonders bevorzugter Weise größer als 100 % ist, bemessenes Aufnahmevolumen von wenigstens 30 ml zur Verfügung, und zwar auch nach insbesondere wiederholter schwallartiger Flüssigkeitsbeaufschlagung im Gebrauch des Hygieneartikels.

Bei der in der beschriebenen Weise extrudierten Flüssigkeitsaufnahme-, -verteiler- und -zwischenspeicherschicht treten die eingangs erörterten Probleme des Zusammenfallens einer Faserstruktur bei Einnässung nicht auf. Nach einer schwallartigen Flüssigkeitsbeaufschlagung wird die Flüssigkeit in den Poren zwischengespeichert und nach und nach an die darunter angeordnete Speicherschicht abgegeben, wo dann eine dauerhafte Speicherung der Flüssigkeit mittels der dort vorgesehenen superabsorbierenden Polymermaterialien stattfindet.

Die Flüssigkeitsaufnahme-, -verteiler- und -zwischenspeicherschicht ist im wesentlichen frei von superabsorbierenden Polymermaterialien, worunter verstanden werden soll, daß sie vorzugsweise weniger als 10, insbesondere weniger als 5 Gew.-%, und besonders bevorzugtermaßen weniger als 1 Gew.-% an superabsorbierenden Polymermaterialien aufweist.

Als thermoplastisches Polymer hat sich in besonders bevorzugter Weise ein Polymer aus der Gruppe der Polyolefine, insbesondere Polypropylen und/oder Polyethylen, erwiesen. Auch entsprechende Copolymere, insbesondere Ethylenvinylacetatcopolymere sowie halogenierte Polyolefine sind verwendbar. Grundsätzlich sind jedoch auch andere thermoplastische Polymere zur Herstellung der erfindungsgemäßen absorbierenden Struktur geeignet, z.B. solche aus der Gruppe der Styrolpolymere.

Es sind Zuschlagstoffe, zu 3 bis 30 Gew.%, insbesondere 10 bis 20 Gew.-%, in Form von Fasern, vorzugsweise Polyesterfasern, vorgesehen. Durch Zusatz von Fasern, deren Schmelz- oder Zersetzungstemperatur höher ist als die Schmelztemperatur des verwendeten thermoplastischen Polymers, werden beim Extrusionsvorgang Kanäle gebildet, die das Eindringen und die Verteilung von wässriger Flüssigkeit in die Struktur und innerhalb der Struktur sowie die Weitergabe der Flüssigkeit an die darunter angeordnete Speicherschicht fördern.

Die Erfindung ermöglicht in besonders vorteilhafter Weise, daß das Flächengewicht, d. h. die Dicke der Flüssigkeitsaufnahme-, -verteiler- und -zwischenspeicherschicht in Längsrichtung und/oder in Querrichtung variiert werden kann, wobei die Längsrichtung definitionsgemäß mit der Extrusionsrichtung übereinstimmen soll. Durch eine entsprechende Gestaltung einer Extrusionsöffnung, insbesondere eines Extrusionsschlitzes, lassen sich an sich beliebige Querschnittsstrukturen erzielen. So könnte insbesondere im Querschnitt, senkrecht zur Längsrichtung betrachtet, die Dicke der absorbierenden Struktur mittig größer sein und entsprechend der Struktur der Extrusionsöffnung zu den Seiten hin in beliebiger Weise abnehmen.

Ebenso kann es sich als vorteilhaft erweisen, wenn die Breite der extrudierten Schicht in Längsrichtung variiert; auf diese Weise lassen sich in der Draufsicht profilierte Schichten erzeugen, wie zum Beispiel eine Sanduhrform.

Die Flüssigkeitsaufnahme-, -verteiler- und -zwischenspeicherschicht kann außerdem eine oberflächenaktive Substanz, insbesondere ein Hydrophilierungsmittel zu einem Anteil von vorzugsweise 0,2 bis 10 Gew.-% umfassen. Die bereits extrudierte Struktur kann dabei sekundär mit dem Hydrophilierungsmittel beaufschlagt werden. In bevorzugter Weise wird dieses Mittel aber gemeinsam mit den übrigen Ausgangsstoffen einer Extrusionseinrichtung zugeführt oder in die bereits erschmolzene Polymermasse injiziert; sie befindet sich also bereits in Mischung mit der Polymerschmelze bevor diese extrudiert wird. Vorteilhafterweise werden hierfür Alkylsulfonate, Fettsäurederivate und Fluorchemikalien - wie sie in der Veröffentlichung "Polymer Melt Additives; Their Chemistry, Structure and Use" (Autoren Gasper et al., Vortrag während der Insight 1999 "Non-wovens Business/Fiber and Fabric Conferences", San Diego, California, 1.-2. November 1999, Proceedings herausgegeben durch Marketing Technology Service, Inc.) beschrieben sind - eingesetzt.

Um die Zugänglichkeit der extrudierten Struktur für wässrige Flüssigkeiten zu erhöhen, ist es vorteilhaft, die extrudierte Struktur einer weiteren mechanischen Behandlung, z. B. einer Streckung, einer Verpressung (Walzung) und/oder einer Perforierung durch ein feines Nadelwerkzeug auszusetzen. Hierdurch kann eine gegebenenfalls abgeschlossene Pore mit anderen Poren in Flüssigkeitskommunikation treten und zum Aufnahmevermögen, aber auch zur Verteilungs- und Weiterleitungsfunktion beitragen bzw. aktiviert werden.

Vorteilhaft ist insbesondere eine mehrstufige Walzung der extrudierten Struktur. Eine mehrstufige Walzung ermöglicht die Anwendung mehrerer Temperatur-und/oder Druckstufen. Damit kann die extrudierte Struktur gezielter hinsichtlich der Erfordernisse ihrer späteren Verwendung verändert/optimiert werden. So hat es sich als vorteilhaft erwiesen, die extrudierte Struktur in einer ersten Kalanderstufe bei einer Temperatur zu verpressen, die geeignet ist, das thermoplastische Polymer in der extrudierten Struktur oberhalb des Erweichungspunktes zu halten. Je nach verwendetem Polymer hat sich eine Temperatur in der Kalanderstufe von 40-90°C, insbesondere 45-70°C, insbesondere 50-60°C als geeignet erwiesen. Vorteilhafterweise kann die extrudierte absorbierende Struktur anschließend in einer zweiten Kalanderstufe verpresst werden, die kalt, insbesondere bei Temperaturen von 0-30 °C, insbesondere bei 15-25°C durchgeführt wird.

Es hat sich ferner als vorteilhaft erwiesen außerdem eine Verstreckung der extrudierten Struktur vorzunehmen.

Es ist auch denkbar, daß die Speicherschicht ebenfalls als extrudierte geschäumte Struktur hergestellt ist. Solchenfalls werden körnige Partikel aus superabsorbierenden Materialien zusammen mit thermoplastischem Polymer in eine Extrusionseinrichtung eingebracht und die thermoplastischen Polymermaterialien werden bei Temperaturen unterhalb der Zersetzungstemperatur der superabsorbierenden Polymermaterialien geschmolzen und zusammen mit diesen extrudiert. Solchenfalls könnten sowohl die Flüssigkeitsaufnahme-, -verteiler- und -zwischenspeicherschicht als auch die Speicherschicht innerhalb der Herstellungsmaschine durch unmittelbare Coextrusion hergestellt werden.

Auch eine auf der körperabgewandten Seite der Speicherschicht vorgesehene flüssigkeitsundurchlässige Folienschicht kann durch Coextrusion mit den vorstehenden Schichten hergestellt sein. Es kann dann vorteilhafterweise auf ein Fixiermittel, wie z. B. einen Heißschmelzkleber, verzichtet werden, da die extrudierten Schichten miteinander, aber auch gegenüber weiteren Lagen und/oder Elementen des Hygieneartikels im Zuge ihrer Herstellung durch Extrusion fixiert werden können.

In vorteilhafter Weiterbildung der Erfindung ist es auch denkbar, daß die Flüssigkeitsaufnahme-, -verteiler- und -zwischenspeicherschicht beidseits in Längsrichtung des Hygieneartikels verlaufende und in Richtung auf den Benutzer emporstehende Wandabschnitte aufweist, die eine Auslaufsperre bilden. Diese Wandabschnitte übernehmen dann die Funktion von in Richtung auf den Benutzer emporstehenden Bündchenelementen, die bei bekannten Hygieneartikeln üblicherweise aus Vliesstoffen mit eingebrachten Elastifizierungsmitteln gebildet sind.

Gegenstand der vorliegenden Erfindung ist auch ein Verfahren nach den Ansprüchen 7-14 zum Herstellen einer Flüssigkeitsaufnahme-, -verteiler- und -zwischenspeicherschicht im Zuge der Herstellung eines Hygieneartikels mit den Merkmalen der Ansprüche 1 bis 6.

Als Treibmittel wird vorzugsweise CO₂ verwendet, wobei gleichwohl auch gesättigte, ungesättigte, cyclische Kohlenwasserstoffe und halogenierte Kohlenwasserstoffe sowie Edelgase wie Argon, Helium, oder Stickstoff oder ein Wasser/Luft-Gemisch oderWaser selbst, vorzugsweise in Form von Feuchtigkeit bei oder in den extrudierten Materialien denkbar wäre.

Es wird vorzugsweise ein solcher Überdruck innerhalb der Extrusionsvorrichtung aufgebaut, daß das Treibmittel sich in einem sogenannten "überkritischen" Zustand befindet, in dem die Phasengrenze zwischen flüssigem und gasförmigem Aggregatzustand verschwindet und lediglich eine einzige homogene Phase vorliegt. Dieser Bereich liegt bei CO₂ bei Temperaturen oberhalb von etwa 31° C und Drücken oberhalb von etwa 73,5 bar vor. In diesem Zustand läßt sich das Treibmittel optimal zur Vorbereitung eines physikalischen Schäumungsvorgangs mit dem geschmolzenen thermoplastischen Polymer vermischen. Wird diese Mischung dann durch eine Extrusionsöffnung in einen Bereich niedrigeren Drucks gegeben, so verdampft das Treibmittel bei abnehmender Temperatur, und es entsteht die geschäumte offenporige Struktur der Flüssigkeitsaufnahme-, -verteiler- und -zwischenspeicherschicht.

Da aber nicht nur ein vorzugsweiser überkritischer Zustand des Treibmittels erreicht werden muß, sondern auch das thermoplastische Polymer zumindest teilerschmolzen werden muß, werden innerhalb der Extrusionsvorrichtung Temperaturen von 80 bis 200° C geschaffen.

Zur Herstellung einer großen Anzahl von entsprechend ausgebildeten Schichtabschnitten erweist es sich als vorteilhaft, wenn der Extrusionsquerschnitt entsprechend oszillierend verändert wird. Dies erfolgt quer zur Extrusionsrichtung, und zwar in der Ablegerichtung, wodurch die Dicke einer extrudierten Bahn variiert wird, oder quer zur Ablegerichtung, wodurch deren Breite variiert wird.

Als ganz besonders vorteilhaft erweist sich, wenn das Extrusionsverfahren unmittelbar in den Herstellungsprozeß für den Hygieneartikel integriert wird und dabei die Flüssigkeitsaufnahme-, -verteiler- und -zwischenspeicherschicht unmittelbar innerhalb einer schnellaufenden Herstellungsmaschine für Hygieneartikel extrudiert wird.

Weitere Einzelheiten, Merkmale und Vorteile der Erfindung ergeben sich aus den beigefügten Patentansprüchen sowie aus der zeichnerischen Darstellung und nachfolgenden Beschreibung eines erfindungsgemäßen Hygieneartikels. In der Zeichnung zeigt:
- Figur 1: eine schematische Ansicht einer Vorrichtung zum Herstellung einer Flüssigkeitsaufnahme-, -verteiler- und -zwischenspeicherschicht für einen erfindungsgemäßen Hygieneartikel;
- Figuren 2 und 3: eine Draufsicht und eine Schnittansicht auf bzw. durch einen Saugkörper eines erfindungsgemäßen Hygieneartikels und
- Figur 4: eine Figur 2 entsprechende Draufsicht auf einen Saugkörper mit in Längsrichtung variierender Breite.

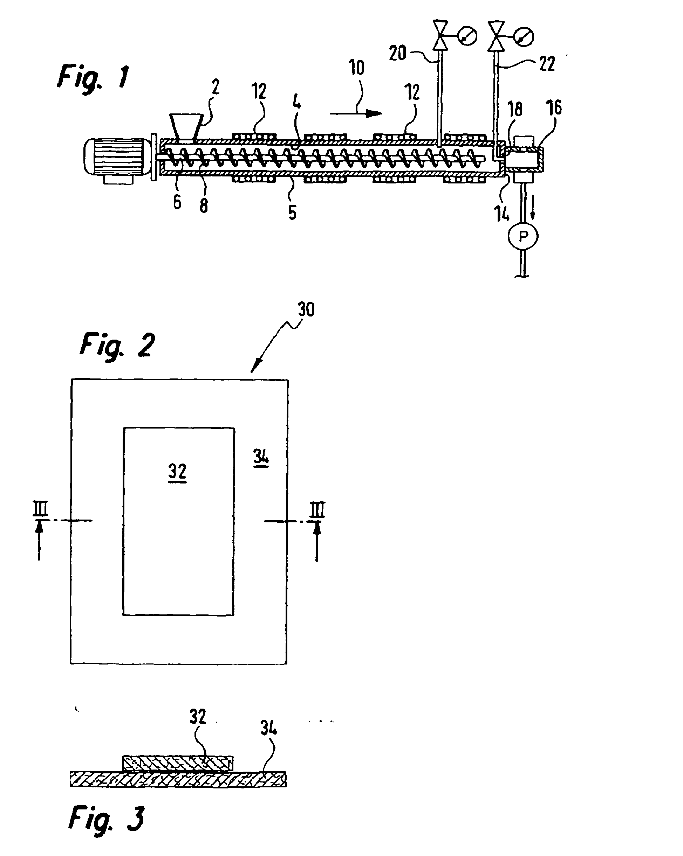

Figur 1 zeigt eine Vorrichtung zum Extrudieren einer Flüssigkeitsaufnahme-, -verteiler- und -zwischenspeicherschicht. Die Vorrichtung umfaßt eine trichterförmige Eingabeeinrichtung 2, über die ein Feststoffgemisch, das vorzugsweise zuvor gemäß der gewichtsprozentualen Zusammensetzung der einzelnen Bestandteile hergestellt wurde, in einen zylindrischen Innenraum 4 eines hochdruckstabilen rohrförmigen Gehäusekörpers 5 der Extrusionsvorrichtung eingegeben eingegeben werden kann. In diesen Innenraum 4 erstreckt sich eine elektromotorisch angetriebene Welle 6 mit einem wendelförmigen Schneckengang 8. Beim Antrieb der Welle 6 wird die eingebrachte Feststoffmischung weiter vermischt und in Längsrichtung 10 gefördert. Am Außenumfang des rohrförmigen Gehäuses 5 sind Heizeinrichtungen 12 vorgesehen.

An dem der Eingabeeinrichtung 2 gegenüberliegenden Ende des rohrförmigen Gehäuses 5 ist an dessen Stirnseite 14 ein Extrusionswerkzeug 16 montierbar. Das Extrusionswerkzeug 16 kommuniziert über eine Öffnung 18 an der Stirnseite 14 mit dem Innenraum 4 des rohrförmigen Gehäuses.

In den Innenraum 4 münden Injektionseinrichtungen 20, 22, wobei die letztere quasi innerhalb der Öffnung 18 mündet. Über die Injektionseinrichtungen 20, 22 kann ein unter Betriebsdruck stehendes Treibmittel in den Innenraum 4 eingebracht werden. Auf diese Weise kann im Innenraum 4 ein Betriebsdruck in Abhängigkeit des im Extrusionsvorgang verwandten Treibmittels, im allgemeinen oberhalb 70 bar, eingestellt und während des Extrusionsvorgangs aufrechterhalten werden.

Zur Herstellung der Flüssigkeitsaufnahme-, -verteiler- und -zwischenspeicherschicht kann beispielsweise als thermoplastisches Polymer ein Polyolefin, insbesondere ein Polypropylen- und/oder Polyäthylen-Granulat, verwendet werden. Dieses Granulat und mit Zuschlagstoffen, in Form von Fasern vermischt.

Das so erhaltene Gemisch wird über die Eingabeeinrichtung 2 in den Innenraum 4 gegeben. Durch die Heizeinrichtungen 12 wird das Gemisch auf eine solche Betriebstemperatur gebracht, daß das thermoplastische Polymer schmilzt.

Über die erwähnten Injektionseinrichtungen 20, 22 wird ein Treibmittel, beispielsweise CO₂, in den Innenraum 4 eingeleitet, so daß dort ein Betriebsdruck herrscht, der zum Extrudieren des teilerschmolzenen Gemischs über das Extrusionswerkzeug 16 geeignet ist. Da das Treibmittel im Zuge der Extrusion zu einer Schäumung des thermoplastischen Polymers führen soll, wird es vorzugsweise im sogenannten "überkritischen Zustand" in den Innenraum 4 eingeleitet.

Beim Durchtritt der so erhaltenen Mischung durch die Extrusionsöffnung des Extrusionswerkzeugs 16 und durch den damit einhergehenden Druckabbau expandiert das Treibmittel und die Mischung wird geschäumt, d. h. es bilden sich durch das expandierende und in der Regel entweichende Treibmittel miteinander kommunizierende Poren oder Hohlräume.

Die Figuren 2 und 3 zeigen eine Draufsicht bzw. eine Schnittansicht auf bzw. durch einen zweischichtigen Saugkörper 30 eines erfindungsgemäßen Hygieneartikels. Der Saugkörper 30 umfaßt eine im Gebrauch körperzugewandte Flüssigkeitsaufnahme-, -verteiler- und -zwischenspeicherschicht 32 und eine auf deren körperabgewandter Seite vorgesehene Speicherschicht 34, die einen Anteil von wenigstens 50 Gew.-%, insbesondere wenigstens 80 Gew.-% an superabsorbierenden Polymermaterialien umfaßt, die beispielsweise in eine Faserstruktur (thermoplastische oder natürliche Fasern) eingebettet sein können.

Die Flüssigkeitsaufnahme-, -verteiler- und -zwischenspeicherschicht ist aus einem durch Extrusion unter Zusatz eines Treibmittels geschäumten thermoplastischen Polymer gebildet. Das Polymer umfaßt Polyethylen und/oder Polyproplyen. Nach der Erfindung sind zwischen 3 und 30 Gew.-% Zuschlagstoffe in Form von thermoplastischen Fasern, vorzugsweise Polyesterfasern, zugesetzt. Zusätzlich können Hydrophilierungsmittel in der Form von Alkylsulfonaten, Fettsäurederivaten oder Fluorchemikalien enthalten sein.

Durch die Extrusion des thermoplastischen Polymers unter Zusatz eines Treibmittels wird eine poröse Schicht erzeugt, die ein Gesamtporenvolumen von wenigstens 30 ml aufweist und sich für die rasche Flüssigkeitsaufnahme, -verteilung und -zwischenspeicherung eignet. Die Längs- und Querabmessungen der Schicht 32 sind geringer als diejenigen der darunter befindlichen Speicherschicht 34 derart, daß die Schicht 32 in der Draufsicht allseitig innerhalb der Speicherschicht 34 angeordnet ist. Auf diese Weise kann auftreffende Flüssigkeit aufgrund der Verteilungswirkung innerhalb der Schicht 32 nicht über die Ränder der Speicherschicht 34 gelangen. Vorzugsweise liegt der Flächenanteil der Schicht 32 bei etwa 55 - 90 % der Fläche der Speicherschicht 34. Es wird aber ausdrücklich darauf hingeweisen, dass auch jede andere geometrische Ausbildung der Schicht 32 denkbar wäre, insbesondere könnte die Schicht 32 in Längsrichtung dieselbe Länge aufweisen wie die Speicherschicht 34, was herstellungstechnisch vorteilhaft wäre.

Figur 4 zeigt eine Draufsicht auf eine weitere Ausführungsform eines Saugkörpers 30, wobei die körperzugewandte Flüssigkeitsaufnahme-, -verteiler- und -zwischenspeicherschicht 32 in Längsrichtung 36, die auch der Extrusionsrichtung entspricht, eine variierende Breite b aufweist. Sie ist im dargestellten Fall sanduhrförmig ausgebildet.

## Patentansprüche

1. Absorbierender Hygieneartikel zum einmaligen Gebrauch, insbesondere Windel, Damenbinde, Inkontinenzvorlage, mit einem wenigstens zweischichtigen Saugkörper (30), der eine im Gebrauch des Artikels körperzugewandte Flüssigkeitsaufnahme-, -verteiler- und -zwischenspeicherschicht (32) und eine auf deren körperabgewandter Seite vorgesehene Speicherschicht (34) mit einem Anteil von wenigstens 50 Gew.-% an superabsorbierenden Polymermaterialien umfasst,
**dadurch gekennzeichnet,**
**dass** die Flüssigkeitsaufnahme-, -verteiler- und -zwischenspeicherschicht (32) aus einem thermoplastischen Polymer unter Zusatz eines Treibmittels extrudiert ist,
**dass** die Flüssigkeitsaufnahme-, -verteiler- und -zwischenspeicherschicht (32) ein Gesamtporenvolumen von wenigstens 30ml aufweist,
**dass** die Flüssigkeitsaufnahme-, -verteiler- und -zwischenspeicherschicht (32) im wesentlichen frei von superabsorbierenden Polymermaterialien ist und dass der Schäumungsgrad größer als 50 % ist,
und **dass** die Flüssigkeitsaufnahme-, -verteiler- und -zwischenspeicherschicht (32) als Zuschlagstoffe 3-30 Gew.-%, insbesondere 10 - 20 Gew.-%, Fasern umfasst.

2. Hygieneartikel nach Anspruch 1, **dadurch gekennzeichnet, dass** das thermoplastische Polymer ein Polyolefin, insbesondere Polypropylen und/oder Polyethylen umfasst.

3. Hygieneartikel nach Anspruch 1 oder 2, **dadurch gekennzeichnet, dass** der Schäumungsgrad größer als 100% ist.

4. Hygieneartikel nach Anspruch 1, 2 oder 3, **dadurch gekennzeichnet, dass** die Fasern von Polyesterfasern gebildet sind.

5. Hygieneartikel nach einem oder mehreren der vorstehenden Ansprüche, **dadurch gekennzeichnet, dass** das Flächengewicht der Flüssigkeitsaufnahme-, -verteiler- und -zwischenspeicherschicht (32) in Längsrichtung und/oder in Querrichtung variiert.

6. Hygieneartikel nach einem oder mehreren der vorstehenden Ansprüche, **dadurch gekennzeichnet, dass** die Breite der Flüssigkeitsaufnahme-, -verteiler- und -zwischenspeicherschicht (32) über deren Längsrichtung variiert.

7. Verfahren zum Herstellen einer Flüssigkeitsaufnahme-, -verteiler- und -zwischenspeicherschicht (32) im Zuge der Herstellung eines Hygieneartikels nach einem oder mehreren der vorstehenden Ansprüche 1-6, die folgenden Verfahrensschritte umfassend:
- Einbringen eines thermoplastischen Polymers in eine Extrusionsvorrichtung,
- Schmelzen des thermoplastischen Polymermaterials,
- Einbringen eines Treibmittels unter Überdruck
- Extrudieren des Gemischs, wobei das Treibmittel bei Druckabbau zur Schäumung des thermoplastischen Polymers führt, wobei als Zuschlagstoff Fasern in die Extrusionsvorrichtung eingebracht werden.

8. Verfahren nach Anspruch 7, **dadurch gekennzeichnet, dass** als Treibmittel CO₂ verwendet wird.

9. Verfahren nach Anspruch 7 oder 8, **dadurch gekennzeichnet, dass** das thermoplastische Polymer bei Temperaturen von 80 - 200 Grad C erschmolzen wird.

10. Verfahren nach einem der Ansprüche 7 - 9, **dadurch gekennzeichnet, dass** als Zuschlagstoff eine oberflächenaktive Substanz in die Extrusionsvorrichtung eingebracht werden.

11. Verfahren nach einem der Ansprüche 7 - 10, **dadurch gekennzeichnet, dass** ein Extrusionsquerschnitt während des Extrudierens verändert wird.

12. Verfahren nach Anspruch 11, **dadurch gekennzeichnet, dass** der Extrusionsquerschnitt oszillierend verändert wird.

13. Verfahren nach Anspruch 7 - 12, **dadurch gekennzeichnet, dass** das Verfahren in einen Herstellungsprozess für Hygieneartikel integriert wird und dabei die Flüssigkeitsaufnahme-, -verteiler- und -zwischenspeicherschicht (32) unmittelbar innerhalb einer schnellaufenden Herstellungsmaschine für Hygieneartikel extrudiert wird.

14. Verfahren nach Anspruch 13, **dadurch gekennzeichnet, dass** innerhalb der schnelllaufenden Herstellungsmaschine die Flüssigkeitsaufnahme-, -verteiler- und -zwischenspeicherschicht (32) und die Speicherschicht durch Coextrusion der Schichten gebildet werden.

## Claims

1. Absorbent sanitary article for single use, in particular a nappy, sanitary towel or incontinence pad, having an at least two-ply absorbent body (30) which comprises a layer (32) facing the body when the article is being used for absorbing, distributing and temporarily storing fluid and a storage layer (34) furnished on the side remote from the body, having a content of at least 50 % by weight of superabsorbent polymer materials, **characterized in that** the layer (32) for absorbing, distributing and temporarily storing fluid is extruded from a thermoplastic polymer with the addition of a blowing agent, and **in that** the layer (32) for absorbing, distributing and temporarily storing fluid has a total pore volume of at least 30 ml, and **in that** the layer (32) for absorbing, distributing and temporarily storing fluid has substantially no superabsorbent polymer materials and **in that** the degree of foaming is greater than 50 % and **in that** the layer (32) for absorbing, distributing and temporarily storing fluid comprises 3 to 30 % by weight, in particular 10 to 20 % by weight, fibres as additive materials.

2. Sanitary article according to claim 1, **characterized in that** the thermoplastic polymer comprises a polyolefin, in particular polypropylene and/or polyethylene.

3. Sanitary article according to claim 1 or 2, **characterized in that** the degree of foaming is greater than 100 %.

4. Sanitary article according to claim 1, 2 or 3, **characterized in that** the fibres are formed from polyester fibres.

5. Sanitary article according to one or more of the preceding claims, **characterized in that** the unit weight of the layer (32) for absorbing, distributing and temporarily storing fluid varies in the longitudinal and/or in the perpendicular direction.

6. Sanitary article according to one or more of the preceding claims, **characterized in that** the width of the layer (32) for absorbing, distributing and temporarily storing fluid varies over its longitudinal direction.

7. Method for producing a layer (32) for absorbing, distributing and temporarily storing fluid as part of producing a sanitary article according to one or more of the preceding claims 1 to 6, comprising the following steps:
- introduction of a thermoplastic polymer into an extrusion apparatus,
- melting the thermoplastic polymer material,
- introduction of a blowing agent under positive pressure,
- extrusion of the mixture, the blowing agent resulting in foaming of the thermoplastic polymer when pressure is reduced, wherein the fibres are introduced into the extrusion apparatus as an additive.

8. Method according to claim 7, **characterized in that** CO₂ is used as the blowing agent.

9. Method according to claim 7 or claim 8, **characterized in that** the thermoplastic polymer is melted at temperatures of 80 to 200 °C.

10. Method according to any one of the claims 7 to 9, **characterized in that** a surfactant substance is introduced into the extrusion apparatus as an additive.

11. Method according to any one of the claims 7 to 10, **characterized in that** an extrusion cross-section is changed during extrusion.

12. Method according to claim 11, **characterized in that** the extrusion cross-section is changed in an oscillating manner.

13. Method according to any one of the claims 7 to 12, **characterized in that** the method is integrated into a process for producing sanitary articles and thereby the layer (32) for absorbing, distributing and temporarily storing fluid is extruded directly inside high-speed machinery for producing sanitary articles.

14. Method according to claim 13, **characterized in that** the layer (32) for absorbing, distributing and temporarily storing fluid and the storage layer are formed inside the high-production machinery by co-extrusion of the layers.

## Revendications

1. Article d'hygiène absorbant à usage unique, en particulier couche, serviette hygiénique, protection pour incontinence, comprenant un corps absorbant constitué d'au moins deux couches (30) qui comprend une couche d'absorption, de répartition et de rétention intermédiaire de liquide (32) orientée vers le corps lors de l'utilisation de l'article et une couche de rétention (34) prévue sur sa face opposée au corps ayant une part de matériaux polymères superabsorbants d'au moins 50 % en poids, **caractérisé en ce que** la couche d'absorption, de répartition et de rétention intermédiaire de liquide (32) est extrudée à partir d'un polymère thermoplastique avec addition d'un agent gonflant, et **en ce que** la couche d'absorption, de répartition et de rétention intermédiaire de liquide (32) présente un volume poreux total d'au moins 30 ml, et **en ce que** la couche d'absorption, de répartition et de rétention intermédiaire de liquide (32) est essentiellement exempte de matériaux polymères superabsorbants, et **en ce que** le taux de moussage est supérieur à 50 % et **en ce que** la couche d'absorption, de répartition et de rétention intermédiaire de liquide (32) comprend comme adjuvants de 3 à 30 % en poids, en particulier de 10 à 20 % en poids de fibres.

2. Article d'hygiène selon la revendication 1, **caractérisé en ce que** le polymère thermoplastique comprend une polyoléfine, en particulier du polypropylène et/ou du polyéthylène.

3. Article d'hygiène selon la revendication 1 ou 2, **caractérisé en ce que** le taux de moussage est supérieur à 100 %.

4. Article d'hygiène selon la revendication 1, 2 ou 3, **caractérisé en ce que** les fibres sont formées par des fibres de polyester.

5. Article d'hygiène selon l'une ou plusieurs des revendications précédentes, **caractérisé en ce que** le poids par unité de surface de la couche d'absorption, de répartition et de rétention intermédiaire de liquide (32) varie dans le sens longitudinal et/ou transversal.

6. Article d'hygiène selon l'une ou plusieurs des revendications précédentes, **caractérisé en ce que** la largeur de la couche d'absorption, de répartition et de rétention intermédiaire de liquide (32) varie sur son sens longitudinal.

7. Procédé de fabrication d'une couche d'absorption, de répartition et de rétention intermédiaire de liquide (32) au cours de la fabrication d'un article d'hygiène selon l'une ou plusieurs des revendications précédentes 1 à 6, comprenant les étapes de procédé suivantes :
- chargement d'un polymère thermoplastique dans un dispositif d'extrusion,
- fusion du matériau polymère thermoplastique,
- chargement d'un agent gonflant sous surpression,
- extrusion du mélange, l'agent gonflant conduisant, lors de la réduction de pression, au moussage du polymère thermoplastique, et des fibres étant chargées dans le dispositif d'extrusion comme adjuvants.

8. Procédé selon la revendication 7, **caractérisé en ce que** du CO₂ est utilisé comme agent gonflant.

9. Procédé selon la revendication 7 ou 8, **caractérisé en ce que** le polymère thermoplastique est fondu à des températures comprises entre 80 et 200 degrés C.

10. Procédé selon l'une des revendications 7 à 9, **caractérisé en ce qu'**une substance tensioactive est chargée dans le dispositif d'extrusion comme adjuvant.

11. Procédé selon l'une des revendications 7 à 10, **caractérisé en ce qu'**une section transversale d'extrusion est modifiée pendant l'extrusion.

12. Procédé selon la revendication 11, **caractérisé en ce que** la section transversale d'extrusion est modifiée de manière oscillante.

13. Procédé selon les revendications 7 à 12, **caractérisé en ce que** le procédé est intégré dans un processus de fabrication d'articles d'hygiène dans lequel la couche d'absorption, de répartition et de rétention intermédiaire de liquide (32) est extrudée directement à l'intérieur d'une machine de fabrication d'articles d'hygiène à cadence rapide.

14. Procédé selon la revendication 13, **caractérisé en ce que** la couche d'absorption, de répartition et de rétention intermédiaire de liquide (32) et la couche de rétention sont formées par co-extrusion des couches à l'intérieur de la machine de fabrication à cadence rapide.
